# EUROPÄISCHE PATENTANMELDUNG

(11) **EP 3 117 773 A1**
(43) Veröffentlichungstag der Anmeldung: **18.01.2017**
(21) Anmeldenummer: 16179147.0
(22) Anmeldetag: 13.07.2016
(51) Int. Cl.: A61B 6/03, A61B 6/04, A61B 6/00

(54) **EINRICHTUNG ZUR MEDIZINISCHEN VERSORGUNG EINES SCHLAGANFALLPATIENTEN**

(30) Priorität: 13.07.2015 DE 102015213031
(71) Anmelder: Neuro-X-Consult GbR, 28357 Bremen (DE)
(72) Erfinder: Papanagiotou, Panagiotis, 28211 Bremen (DE); Roth, Christian, 28357 Bremen (DE)
(74) Vertreter: Eisenführ Speiser

(57) **Zusammenfassung**

Die Erfindung betrifft eine Einrichtung (1) zur medizinischen Versorgung eines Schlaganfallpatienten, mit einer innerhalb eines Diagnosebereichs (9) angeordneten Diagnoseeinheit (3) zum Diagnostizieren eines Schlaganfalls bei dem Schlaganfallpatienten und einer innerhalb eines Behandlungsbereichs (11) angeordneten Behandlungseinheit (5) zur transarteriellen Behandlung des Schlaganfallpatienten, wobei der Diagnosebereich (9) und der Behandlungsbereich (11) benachbart zueinander angeordnet sind.

## Beschreibung

Die Erfindung betrifft eine Einrichtung zur medizinischen Versorgung eines Schlaganfallpatienten, mit einer innerhalb eines Diagnosebereichs angeordneten Diagnoseeinheit zum Diagnostizieren eines Schlaganfalls bei dem Schiaganfallpatienten und einer innerhalb eines Behandlungsbereichs angeordneten Behandlungseinheit zur transarteriellen Behandlung des Schiaganfallpatienten.

Der Schlaganfall ist die häufigste Ursache einer permanenten Behinderung, die zweithäufigste Ursache einer Demenz und die dritthäufigste Todesursache in den westlichen Industrieländern. Nach Schätzungen der WHO sterben jährlich ungefähr 5,7 Millionen Menschen an den Folgen eines Schlaganfalls. In Deutschland erleiden jährlich ungefähr 200.000 Menschen einen ersten Schlaganfall.

Bei den Überlebenden nach einem Schlaganfall sind häufig die Rehabilitationskosten exorbitant hoch, durch bestehende Restbehinderung sind die Menschen häufig durch ein vermindertes Einkommen und geminderte Produktivität sowie durch Einschränkungen in ihren täglichen sozialen Aktivitäten belastet. Zwischen 2012 und 2030 ist zu erwarten, dass die direkten medizinischen Schlaganfall-assoziierten Kosten sich verdreifachen werden. Dies resultiert durch die steigende Inzidenz und Prävalenz des Schlaganfalls aufgrund des Alterns der Bevtilkerung.

Die rechtzeitige und effektive Behandlung in der Akutphase des Schlaganfalls kann Hirngewebe retten und somit die Langzeitfoigen minimieren mit einer Reduzierung der assoziierten Morbidität und Mortalität. Gerade in den ersten Stunden nach einem Schlaganfall ist die Durchblutungsstörung teilweise reversibel. Wenn das verschlossene Gefäß durch eine Medikamentenbehandlung (Thrombolyse) oder durch einen Kathetereingriff (Thrombektomie) wieder eröffnet werden kann, kann der Schaden minimiert werden.

In den letzten Monaten zeigten neuere Studien, dass auch Patienten mit einem besonders schwerwiegenden Schlaganfall und einem zentralen Hirnarterienverschluss von hochspeziaüsierten interventionellen Techniken profitieren können. Von Dezember 2014 bis April 2015 zeigten 5 multizentrische randomisierte klinische Studien die Effektivität der endovaskulären Behandlung (Thrombektomie) bei Patienten mit einem akuten Schlaganfall durch einen Verschluss einer Hirnarterie.

Während jeder Minute, in der ein akuter Schlaganfall unbehandelt bleibt, verliert der Patient im Mittelwert 1,9 Millionen Neuronen, 13,8 Billionen Synapsen und 12 Kilometer axonale Fasern. Diese Fakten machen den ischämischen Schlaganfall zu einem der wichtigsten Notfälle in der Medizin. Für die Patienten, die einen akuten Schlaganfall erleiden, sowie auch für die Ärzte und das Pflegepersonal, die in die Behandlung involviert sind, zählt jede Sekunde, daher der Ausdruck "Time is brain".

In der Vergangenheit wurden die Verfahren und die eingesetzten medizinischen Geräte, welche zum Diagnostizieren eines Schlaganfalls Verwendung finden, kontinuierlich weiterentwickelt und somit die benötigte Zeit zum Diagnostizieren eines Schlaganfalls reduziert. Eine analoge Entwicklung hat in der Vergangenheit auch im Hinblick auf die Verfahren und eingesetzten medizinischen Geräte zur Behandlung von Schlaganfallpatienten stattgefunden.

So werden bei einer Akutbehandlung von ischämischen Schlaganfallpatienten, bei welchen Einengungen oder Verschlüsse der hirnversorgenden Arterien bestehen zusätzlich zur intravenösen Thrombolyse transarterielle Eingriffe durchgeführt, im Rahmen derer die Einengungen beseitigt werden und die den Verschlüssen zugrunde liegenden Thromben mittels mechanischer Thrombektomie entfernt werden.

Bei transarteriellen Eingriffen werden beispielsweise Katheter über die Leistenarterie bis zu dem verschlossenen Gefäß vorgeführt und hierüber entweder das Blutgerinnsel welches die Ader verstopft abgesaugt oder mittels eines Drahtgeflechtes entfernt. Jedoch bestehen in medizinischen Versorgungszentren regelmäßig Organisations- und Ausstattungsstrukturen, weiche eine Akutbehandlung von Schlaganfallpatienten beeinträchtigen können. So ist festzustellen, dass die notwendigen medizinischen Geräte zum Diagnostizieren eines Schlaganfalls und zum Behandeln eines Schlaganfallpatienten auf unterschiedlichen Stationen, teilweise sogar in verschiedenen Fachkliniken der bekannten Versorgungszentrumen positioniert sind. Hieraus resultiert ein zusätzlicher Zeitaufwand, um den Schlaganfallpatienten nach dem Diagnostizieren des Schlaganfalls auf diejenige Station oder die Fachklinik des Versorgungszentrums zu transportieren, in welcher die Akutbehandlung des Schlaganfallpatienten erfolgt.

Ferner werden die behandelnden Ärzte in medizinischen Versorgungszentren regelmäßig mit dem Problem konfrontiert, dass die Diagnoseeinheit zum Diagnostizieren eines Schlaganfalls und/oder die Behandlungseinheit zur Behandlung des Schlaganfallpatienten aufgrund von Patientenuntersuchungen belegt sind. Dies ist darauf zurückzuführen, dass die Diagnose- und Behandlungseinheiten, welche sich zur medizinischen Versorgung von Schlaganfallpatienten eigenen, ebenfalls auch zur Diagnose und Behandlung anderer Krankheiten verwendet werden. Eine derartige Verwendung von vorhandenen Diagnose- und Behandlungseinheiten steigert zwar die Wirtschaftlichkeit der Gerätenutzung, führt jedoch im Hinblick auf die Versorgung von Schlaganfallpatienten zu einer häufig unnötigen Diagnose- und/oder Behandlungsverzögerung, aus welcher der Schlaganfallpatient schwerwiegende gesundheitliche Schäden erleiden kann.

Die Aufgabe der Erfindung ist es somit eine Möglichkeit zu schaffen, die medizinische Versorgung von Schlaganfallpatienten zu beschleunigen.

Die Aufgabe wird mit einer Einrichtung der eingangs genannten Art gelöst, wobei der Diagnosebereich und der Behandlungsbereich benachbart zueinander angeordnet sind.

Die Erfindung erreicht eine Beschleunigung der medizinischen Versorgung von Schlaganfallpatienten auf überraschend einfache Weise dadurch, dass die Zeit, welche der Transport des Schlaganfallpatienten von der Diagnoseeinheit innerhalb des Diagnosebereichs zu der Behandlungseinheit innerhalb eines Behandlungsbereichs in Anspruch nimmt, erheblich reduziert wird. Diese Zeitersparnis führt zur Vermeidung oder zumindest zu einer Reduzierung der Nervenzellenschädigung und kann den Schlaganfallpatienten vor irreparablen gesundheitlichen Schäden bewahren.

In einer vorteilhaften Ausführungsform der erfindungsgemäßen Einrichtung sind die Diagnoseeinheit und die Behandlungseinheit in einem gemeinsamen Versorgungsraum angeordnet. Durch die Anordnung der Diagnoseeinheit und der Behandlungseinheit innerhalb eines gemeinsamen Versorgungsraumes wird ein oftmals zeitaufwändiger Transport des Schlaganfallpatienten zwischen verschiedenen Räumen, welche beispielsweise durch enge Raumübergänge miteinander verbunden sind und die manuelle Betätigung einer Raumtrenntür erfordern, vermieden. Die Versorgungszeit des Schlaganfallpatienten wird somit weiter reduziert, wodurch gesundheitliche Schäden bei dem Schlaganfallpatienten vermieden werden können. Ein Raum ist dabei ein gegenüber einem Flur- oder Gangbereich abgegrenztes Zimmer, beispielsweise innerhalb eines medizinischen Versorgungszentrums, welches über einen Eingang erreicht werden kann und durch Seitenwände begrenzt wird.

Der Diagnosebereich und der Behandlungsbereich sind vorzugsweise jeweils optisch eingrenzbar, beispielsweise durch einen oder mehrere modulare Bereichstrenner. Derartige Bereichstrenner sind vorzugsweise als mobile Wandelemente ausgebildet, welche insbesondere zur Erweiterung miteinander koppelbar sind.

In einer vorteilhaften Ausführungsform der erfindungsgemäßen Einrichtung ist der Diagnosebereich in einem ersten Versorgungsraum angeordnet und der Behandlungsbereich ist in einem zweiten Versorgungsraum angeordnet, wobei der erste Versorgungsraum und der zweite Versorgungsraum benachbart zueinander angeordnet sind. Auf diese Weise wird eine räumlich-strukturelle Entkopplung des Diagnosebereichs und des Behandlungsbereichs herbeigeführt. Hierdurch kann der Diagnosebereich und/oder der Behandlungsbereich nach erfolgter Diagnose oder Behandlung bereits für die medizinische Versorgung eines neuen Schlaganfallpatienten vorbereitet werden, ohne dass es zur Beeinträchtigung der Diagnose oder Behandlung in dem jeweils anderen Bereich kommt. Somit kann die Einrichtung in Ausnahmesituationen zur schnelleren medizinischen Versorgung mehrerer Schlaganfallpatienten genutzt werden.

Die erfindungsgemäße Einrichtung wird durch eine Zwischentür weitergebildet, welche zwischen dem Diagnosebereich und dem Behandlungsbereich angeordnet ist. Die Zwischentür ist vorzugsweise als automatische Schiebetür ausgebildet. Mittels der Zwischentür ist der Diagnosebereich von dem Behandlungsbereich abtrennbar. Eine Abtrennung des Diagnosebereichs von dem Behandlungsbereich erlaubt es beispielsweise bei Belegung des Behandlungsbereichs den Diagnosebereich bereits für den nächsten Patienten nutzbar zu machen.

Sind der Diagnosebereich und der Behandlungsbereich in einem gemeinsamen Versorgungsraum angeordnet, dient die Zwischentür als Bereichstrenner. Vorzugsweise befindet sich die Zwischentür bei dieser Ausführungsform während der medizinischen Versorgung eines Schlaganfallpatienten in einem geöffneten Zustand, sodass der Transport des Schlaganfallpatienten von dem Diagnosebereich in den Behandlungsbereich nicht durch die Zwischentür beeinträchtigt wird. Die Zwischentür befindet sich vorzugsweise in einem geschlossenen Zustand, wenn der Diagnosebereich oder der Behandlungsbereich zur medizinischen Versorgung eines Patienten genutzt wird und die medizinische Versorgung einen Transport des Patienten in den jeweils anderen Bereich nicht erfordert.

Ist der Diagnosebereich in einem ersten Versorgungsraum und der Behandlungsbereich in einem zweiten Versorgungsraum angeordnet, dient die Zwischentür vorzugsweise als Raumtrenner. Vorzugsweise ist die Schiebetür mit einer Betätigungseinrichtung und einem Bewegungssensor gekoppelt und derart gesteuert, dass bei Sensierung einer Bewegung im Bereich der Zwischentür, beispielsweise im Umkreis von 1m, die Betätigungseinrichtung die Zwischentür öffnet.

In einer bevorzugten Ausführungsform der erfindungsgemäßen Einrichtung umfasst die Diagnoseeinheit einen Computertomographen. Mittels des Computertomographen kann der behandelnde Arzt feststellen, ob es sich um einen durch eine Blutung oder einen Gefäßverschluss (Ischämie) hervorgerufenen Schlaganfall handelt. Auf Grundlage dieser ersten Erkenntnis können im Folgenden weitere Diagnose- und Behandlungsmaßnahmen festgelegt werden. Computertomographen sind mit einer Vielzahl unterschiedlicher Geräteeigenschaften verfügbar und eigenen sich aufgrund der vielfältigen Einstellmöglichkeiten für die Akutdiagnose von Schlaganfällen bei verschiedenen Patiententypen.

In einer weiteren vorteilhaften Ausführungsform der erfindungsgemäßen Einrichtung ist der Computertomograph ausgewählt aus der Gruppe bestehend aus: Spiral-Computertomograph, Helix-Computertomograph, Mehrzeilen-Computertomograph, Dual-Source-Computertomograph, Zwei-Spektren-Computertomograph und Multi-Energy-Computertomograph.

Bereits Mehrzeilen-Computertomographen bieten einen ausreichenden Leistungsumfiang, um bei der Diagnose von Schlaganfällen eingesetzt zu werden und stellen somit aufgrund ihres vergleichsweise geringen Anschaffungspreises einen bevorzugten Computertomographentypen dar. Ferner können auch die übrigen Computertomographen, deren Leistungsumfang üblicherweise weitere Diagnosemöglichkeiten umfasst, bei der Diagnose von Schlaganfällen Anwendung finden. Somit kann der verwendete Computertomograph beispielsweise nach den in dem jeweiligen Gesundheitszentrum zusätzlich angebotenen Diagnoseverfahren ausgewählt werden.

Die erfindungsgemäße Einrichtung wird ferner dadurch vorteilhaft weitergebildet, dass die Behandlungseinheit zur Durchführung einer minimal-invasiven Katheterintervention und vorzugsweise zur Durchführung einer Angiographie bei dem Schlaganfallpatienten eingerichtet ist. Mittels der Angiografie können die Blutgefäße und Blutströme im Gehirn dargestellt werden. Bei der Angiographie wird dem Schlaganfallpatienten intraarteriell ein Kontrastmittel verabreicht. Somit lässt sich die Position der Verengung oder des Verschlusses zuverlässig und präzise bestimmen, sodass die Behandlung, beispielsweise die transarterielle minimal-invasive Katheterintervention zügig und mit hoher Genauigkeit ausgeführt werden kann. Durch den minimal-invasiven Eingriff kommt es lediglich zu kleinen Verletzungen der Haut und der Gefäße des Schlaganfallpatienten. Bei der transarteriellen Katheterintervention wird ein Katheter an einem anatomisch zugänglichen Bereich, beispielsweise in der Leistengegend, in die betroffene Arterie des Schlaganfallpatienten eingebracht und durch die Arterie zu der Position geschoben, an der sich die Verengung oder der Verschluss befindet. Die Katheterintervention stellt ein sicheres Behandlungsverfahren mit einem geringen Behandlungsrisiko für den Schlaganfallpatienten dar und erlaubt ein zuverlässiges Auflösen von Durchblutungsstörungen, welche durch Verengungen oder Verschlüsse hervorgerufen werden.

Es ist ferner eine erfindungsgemäße Einrichtung bevorzugt, bei welcher die Behandlungseinheit eine Anlage zur Durchführung einer biplanaren digitalen Subtraktionsangiographie aufweist. Bei der Subtraktionsangiografie wird ein vor der Injektion des Kontrastmittels aufgenommenes Bild der betroffenen Körperregionen mittels eines Computers mit einem nach der Injektion des Kontrastmittels aufgenommenen Bild abgezogen. Auf diese Weise können besonders kontrastreiche Aufnahmen erreicht werden, wodurch die Behandlung schneller und präziser erfolgen kann.

Die erfindungsgemäße Einrichtung weist vorzugsweise eine Steuerungseinheit zur Steuerung der Diagnoseeinheit und/oder der Behandlungseinheit auf, wobei die Steuerungseinheit außerhalb des Diagnosebereichs und/oder des Behandlungsbereichs angeordnet ist. Insbesondere ist die Steuerungseinheit als gemeinsame Steuerungseinheit für die Diagnoseeinheit und die Behandlungseinheit ausgebildet. Vorzugsweise ist die Steuerungseinheit innerhalb des gemeinsamen Versorgungsraums oder alternativ in einem weiteren Versorgungsraum angeordnet, wobei der weitere Versorgungsraum benachbart zu dem gemeinsamen Versorgungsraum oder dem ersten und/oder zweiten Versorgungsraum angeordnet ist. Durch die Anordnung der Steuerungseinheit außerhalb des Diagnosebereichs und/oder des Behandlungsbereichs wird die Steuerung der Diagnoseeinheit und/oder der Behandlungseinheit örtlich von der unmittelbaren Patientenumgebung entkoppelt Medizinische Maßnahmen, welche von dem behandelnden Arzt am Schlaganfallpatienten durchgeführt werden, können somit unter größter Konzentration und ohne Ablenkung erfolgen. Außerdem wird durch die Anordnung der Steuerungseinheit außerhalb des Diagnosebereichs und/oder des Behandlungsbereichs das Kontaminationsrisiko innerhalb dieser Bereiche erheblich reduziert. Auch die Steuerung der Diagnoseeinheit und/oder Behandlungseinheit kann durch eine solche räumliche Entkopplung ohne Ablenkung durch medizinische Maßnahmen erfolgen, weiche gleichzeitig unmittelbar am Patienten durchgeführt werden. Vorzugsweise umfasst die Steuerungseinheit ein Steuerungsgerät zur Steuerung der Diagnoseeinheit und ein Steuerungsgerät zur Steuerung der Behandlungseinheit.

Die erfindungsgemäße Einrichtung wird ferner weitergebildet durch eine Auswerteinrichtung zur Auswertung von Daten, welche von der Diagnoseeinheit und/oder der Behandlungseinheit zur Verfügung gestellt werden. Die Auswerteinrichtung ist ebenfalls außerhalb des Diagnosebereichs und/oder des Behandlungsbereichs angeordnet. Vorzugsweise ist die Auswerteinrichtung innerhalb des gemeinsamen Versorgungsraums oder alternativ in einem weiteren Versorgungsraum angeordnet, wobei der weitere Versorgungsraum benachbart zu dem gemeinsamen Versorgungsraum oder dem ersten und/oder zweiten Versorgungsraum angeordnet ist. Durch die Anordnung der Auswerteinrichtung außerhalb des Diagnosebereichs und/oder des Behandlungsbereichs wird die Auswertung der Daten, welche von der Diagnoseeinheit und/oder der Behandlungseinheit zur Verfügung gestellt werden, örtlich von der unmittelbaren Patientenumgebung entkoppelt. Medizinische Maßnahmen, weiche von dem behandelnden Arzt am Schlaganfallpatienten durchgeführt werden, können somit unter größter Konzentration und ohne Ablenkung erfolgen. Vorzugsweise umfasst die Auswerteinrichtung ein Auswertmodul zur Auswertung der Daten, welche von der Diagnoseeinheit zur Verfügung gestellt werden, und ein Auswertmodul zur Auswertung der Daten, welche von der Behandlungseinheit zur Verfügung gestellt werden. Die Steuerungseinheit und die Auswerteinrichtung sind vorzugsweise in einem gemeinsamen Kontrollbereich angeordnet.

Eine besonders bevorzugte Ausführungsform der erfindungsgemäßen Einrichtung weist einen Untersuchungstisch auf, welcher sowohl mit der Diagnoseeinheit als auch mit der Therapieeinheit koppelbar ist. Somit wird eine aufwändige Umlagerung des Patienten vermieden und der Transport zwischen den Bereichen kann schneller und effizienter erfolgen.

Eine weitere Ausführungsform der erfindungsgemäßen Einrichtung weist eine Transporteinrichtung zum Transportieren des Schlaganfallpatienten von der Diagnoseeinheit zu der Behandlungseinheit auf. Vorzugsweise ist die Transporteinrichtung an die Diagnoseeinheit und/oder die Behandlungseinheit angepasst und erlaubt das Diagnostizieren des Schlaganfalls unter Verwendung der Diagnoseeinheit und/oder die Behandlung des Schlagfallpatienten unter Verwendung der Behandlungseinheit, währenddessen sich der Schlaganfallpatient auf der Transporteinrichtung befindet.

In einer vorteilhaften Ausführungsform der erfindungsgemäßen Einrichtung umfasst die Transporteinrichtung eine Patientenliege, welche zwischen einer Diagnoseposition und einer Behandlungsposition verfahrbar ist, wobei in der Diagnoseposition der Schlaganfall bei dem Schlaganfallpatienten mittels der Diagnoseeinheit diagnostizierbar ist und in der Behandlungsposition der Schlaganfallpatient mittels der Behandlungseinheit behandelbar ist. Insbesondere weist die Patientenliege einen oder mehrere Verstellmechanismen auf, mittels welchen die Position und/oder Lage der Schlaganfallpatienten verändert werden kann. Beispielsweise umfasst ein erster Verstellmechanismus eine Höhenverstelleinrichtung, mittels welcher die Liegeposition des Schlaganfallpatienten erhöht oder herabgesetzt werden kann. Vorzugsweise umfasst der erste oder ein alternativer oder zusätzlicher zweiter Verstellmechanismus eine Neigungsverstelleinrichtung, mittels welcher die Neigung des Schlaganfallpatienten verändert werden kann. Somit lässt sich die Patientenliege an verschiedene Patiententypen anpassen und erlaubt eine rasche Erstkonfiguration bevor es zum Einsatz der Diagnoseeinheit und der Behandlungseinheit kommt.

Bevorzugt ist ferner eine erfindungsgemäße Einrichtung, bei welcher die Transporteinrichtung dazu eingerichtet ist, den Schlaganfallpatienten entlang eines vorbestimmten Transportpfades zu transportieren. Der Transportpfad erstreckt sich vorzugsweise von der Diagnoseposition zu der Behandlungsposition. Durch die Vorgabe eines vorbestimmten Transportpfades wird das Risiko eines unsachgemäßen oder nachteiligen Patiententransports von der Diagnoseeinheit zu der Behandlungseinheit reduziert. Gleichzeitig wird die Transportzeit verkürzt.

Vorzugsweise weist die Transporteinrichtung eine sich entlang des Transportpfades erstreckende Transportschiene auf, welche mit der Patientenliege gekoppelt ist und diese entlang des Transportpfades führt.

Eine weitere bevorzugte Ausführungsform der erfindungsgemäßen Einrichtung weist zumindest ein Narkosegerät, insbesondere ein mobiles Narkosegerät, zum Narkotisieren des Schlaganfallpatienten auf. Vorzugsweise ist das Narkosegerät innerhalb des Diagnosebereichs oder des Behandlungsbereichs angeordnet. Bevorzugt ist ferner eine innerhalb des Diagnosebereichs oder des Behandlungsbereichs angeordnete Einrichtung zur Durchführung einer Bronchialabsaugung. Insbesondere sind in dem Diagnosebereich, dem Behandlungsbereich und/oder dem Kontrollbereich ein oder mehrere Monitore angeordnet, welche dazu eingerichtet sind, diagnoserelevante und/oder untersuchungsrelevante Informationen bereitzustellen.

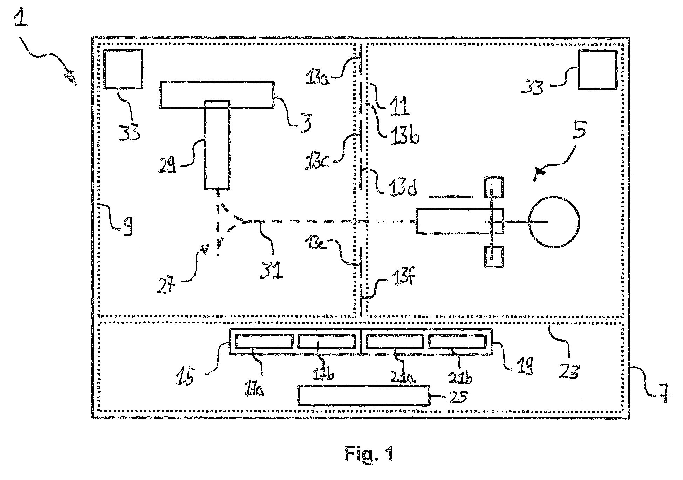

Im Folgenden wird die Erfindung anhand eines bevorzugten Ausführungsbeispiels unter Bezugnahme auf die beiliegende Zeichnung näher erläutert. Die Figur zeigt:
eine Ausführungsform der erfindungsgemäßen Einrichtung in einer schematischen Darstellung.

Gemäß der Figur umfasst die Einrichtung 1 zur medizinischen Versorgung eines Schlaganfallpatienten eine Diagnoseeinheit 3 zum Diagnostizieren eines Schlaganfalls bei dem Schlaganfallpatienten und eine Behandlungseinheit 5 zur transarteriellen Behandlung des Schlaganfallpatienten.

Die Behandlungseinheit 5 ist zur Durchführung einer Angiographie und einer minimalinvasiven Katheterintervention bei dem Schlaganfallpatienten eingerichtet. Die Diagnoseeinheit 3 umfasst einen Computertomographen, weicher als Multi-Energy-Computertomograph ausgebildet ist.

Die Diagnoseeinheit 3 und die Behandlungseinheit 5 sind in einem gemeinsamen Versorgungsraum 7 angeordnet, wobei die Diagnoseeinheit 3 in einem Diagnosebereich 9 des Versorgungsraums 7 angeordnet ist und die Behandlungseinheit 5 beabstandet von der Diagnoseeinheit 3 in einem Behandlungsbereich 11 des Versorgungsraums 7 angeordnet ist. Der Diagnosebereich 9 und der Behandlungsbereich 11 grenzen unmittelbar aneinander an. Zwischen dem Diagnosebereich 9 und dem Behandlungsbereich 11 sind mehrere als mobile Wandelemente ausgebildete Bereichstrenner 13a-13f angeordnet.

In einem außerhalb des Diagnosebereichs 9 und des Behandlungsbereichs 11 liegenden Kontrollbereich 23 sind eine Steuerungseinheit 15 und eine Auswerteinrichtung 19 angeordnet. Die Steuerungseinheit 15 ist zur Steuerung der Diagnoseeinheit 3 und der Behandlungseinheit 5 eingerichtet. Die Steuerungseinheit 15 umfasst ein Steuerungsgerät 17a zur Steuerung der Diagnoseeinheit 3 und ein Steuerungsgerät 17b zur Steuerung der Behandlungseinheit 5. Die Auswerteinrichtung 19 ist zur Auswertung von Daten eingerichtet, welche von der Diagnoseeinheit 3 und der Behandlungseinheit 5 zur Verfügung gestellt werden. Die Auswerteinrichtung 19 umfasst ein Auswertmodul 21a zur Auswertung der Daten, welche von der Diagnoseeinheit 3 zur Verfügung gestellt werden, und ein Auswertmodul 21b zur Auswertung der Daten, welche von der Behandlungseinheit 5 zur Verfügung gestellt werden.

Die Einrichtung 1 weist ferner eine Transporteinrichtung 27 zum Transportieren des Schlaganfallpatienten von der Diagnoseeinheit 3 zu der Behandlungseinheit 5 auf. Die Transporteinrichtung 27 umfasst eine Patientenliege 29 und ein Schienensystem, welches dazu eingerichtet ist, die Patientenliege 29 samt dem Schlaganfalipatienten entlang eines vorbestimmten Transportpfades 31 zu transportieren.

Die Patientenliege 27 ist zwischen einer Diagnoseposition und einer Behandlungsposition verfahrbar, wobei in der Diagnoseposition der Schlaganfall bei dem Schlaganfallpatienten mittels der Diagnoseeinheit 3 diagnostizierbar ist und in der Behandlungsposition der Schlaganfallpatient mittels der Behandlungseinheit 5 behandelbar ist.

In dem Diagnosebereich 9 und dem Behandlungsbereich 11 ist jeweils ein mobiles Narkosegerät 33 zum Narkotisieren des Schlaganfallpatienten angeordnet. Ferner ist in dem Kontrollbereich weitere Labordiagnostik 25 zur medizinischen Versorgung des Schlaganfallpatienten angeordnet, insbesondere zur Bestimmung eines Wertes oder mehrerer Werte ausgewählt aus der Gruppe bestehend aus: Thrombozytenzahl, Leukozytenzahl, Erythrozytenzahl, c-glutamyltransferase, p-amylase, Glukose, aPTT, INR.

### Bezugszeichenliste

- 1: Einrichtung zur medizinischen Versorgung eines Schlaganfallpatienten
- 3: Diagnoseeinheit
- 5: Behandlungseinheit
- 7: Versorgungsraum
- 9: Diagnosebereich
- 11: Behandlungsbereich
- 13a -13f: Bereichstrenner
- 15: Steuerungseinheit
- 17a, 17b: Steuerungsgeräte
- 19: Auswerteinrichtung
- 21a, 21b: Auswertmodule
- 23: Kontrollbereich
- 25: Labordiagnostik
- 27: Transporteinrichtung
- 29: Patientenliege
- 31: Transportpfad
- 33: Narkosegerät

## Patentansprüche

1. Einrichtung (1) zur medizinischen Versorgung eines Schlaganfallpatienten, mit
- einer innerhalb eines Diagnosebereichs (9) angeordneten Diagnoseeinheit (3) zum Diagnostizieren eines Schlaganfalls bei dem Schlaganfallpatienten, und
- einer innerhalb eines Behandlungsbereichs (11) angeordneten Behandlungseinheit (5) zur transarteriellen Behandlung des Schlaganfallpatienten,
**dadurch gekennzeichnet, dass** der Diagnosebereich (9) und der Behandlungsbereich (11) benachbart zueinander angeordnet sind.

2. Einrichtung (1) nach Anspruch 1,
**dadurch gekennzeichnet, dass** der Diagnosebereich (9) und der Behandlungsbereich (11) in einem gemeinsamen Versorgungsraum (7) angeordnet sind.

3. Einrichtung (1) nach Anspruch 1,
**dadurch gekennzeichnet, dass** der Diagnosebereich (9) in einem ersten Versorgungsraum angeordnet ist und der Behandlungsbereich (11) in einem zweiten Versorgungsraum angeordnet ist, wobei der erste Versorgungsraum und der zweite Versorgungsraum benachbart zueinander angeordnet sind.

4. Einrichtung (1) nach einem der vorstehenden Ansprüche,
**gekennzeichnet durch** eine Zwischentür, welche zwischen dem Diagnosebereich (9) und dem Behandlungsbereich (11) angeordnet ist.

5. Einrichtung (1) nach einem der vorstehenden Ansprüche,
**dadurch gekennzeichnet, dass** die Diagnoseeinheit (3) einen Computertomographen umfasst.

6. Einrichtung (1) nach Anspruch 5,
**dadurch gekennzeichnet, dass** der Computertomograph ausgewählt ist aus der Gruppe bestehend aus: Spiral-Computertomograph, Helix-Computertomograph, Mehrzeilen-Computertomograph, Dual-Source-Computertomograph, Zwei-Spektren-Computertomograph und Multi-Energy-Computertomograph.

7. Einrichtung (1) nach einem der vorstehenden Ansprüche,
**dadurch gekennzeichnet, dass** die Behandlungseinheit (5) zur Durchführung einer minimal-invasiven Katheterintervention und vorzugsweise zur Durchführung einer Angiographie bei dem Schlaganfallpatienten eingerichtet ist.

8. Einrichtung (1) nach Anspruch 7,
**dadurch gekennzeichnet, dass** die Behandlungseinheit (5) eine Anlage zur Durchführung einer biplanaren digitalen Subtraktionsangiographie aufweist.

9. Einrichtung (1) nach einem der vorstehenden Ansprüche,
**gekennzeichnet durch** eine Steuerungseinheit (15) zur Steuerung der Diagnoseeinheit (3) und/oder der Behandlungseinheit (5), wobei die Steuerungseinheit (15) vorzugsweise außerhalb des Diagnosebereichs (9) und/oder des Behandiungsbereichs (11) angeordnet ist.

10. Einrichtung (1) nach einem der vorstehenden Ansprüche,
**gekennzeichnet durch** eine Auswerteinrichtung (19) zur Auswertung von Daten, welche von der Diagnoseeinheit (3) und/oder der Behandlungseinheit (5) zur Verfügung gestellt werden, wobei die Auswerteinrichtung (19) vorzugsweise außerhalb des Diagnosebereichs (9) und/oder des Behandlungsbereichs (11) angeordnet ist.

11. Einrichtung (1) nach einem der vorstehenden Ansprüche,
**gekennzeichnet durch** eine Transporteinrichtung (27) zum Transportieren des Schlaganfallpatienten von der Diagnoseeinheit (3) zu der Behandlungseinheit (5).

12. Einrichtung (1) nach Anspruch 11,
**dadurch gekennzeichnet, dass** die Transporteinrichtung (27) eine Patientenliege (29) umfasst, welche zwischen einer Diagnoseposition und einer Behandlungsposition verfahrbar ist, wobei in der Diagnoseposition der Schlaganfall bei dem Schlaganfallpatienten mittels der Diagnoseeinheit (3) diagnostizierbar ist und in der Behandlungsposition der Schlaganfallpatient mittels der Behandlungseinheit (5) behandelbar ist.

13. Einrichtung (1) nach Anspruch 11 oder 12,
**dadurch gekennzeichnet, dass** die Transporteinrichtung (27) dazu eingerichtet ist, den Schlaganfallpatienten entlang eines vorbestimmten Transportpfades (31) zu transportieren.

14. Einrichtung (1) nach Anspruch 13,
**dadurch gekennzeichnet, dass** die Transporteinrichtung (27) eine sich entlang des Transportpfades (31) erstreckende Transportschiene aufweist, welche mit der Patientenliege (29) gekoppelt ist und diese entlang des Transportpfades (31) führt.

15. Einrichtung (1) nach einem der vorstehenden Ansprüche,
**gekennzeichnet durch** zumindest ein Narkosegerät (33), insbesondere ein mobiles Narkosegerät, zum Narkotisieren des Schlaganfallpatienten, wobei das Narkosegerät innerhalb des Diagnosebereichs (9) oder des Behandlungsbereichs (11) angeordnet ist.
